# EUROPEAN PATENT SPECIFICATION

(11) **EP 0 462 466 B1**
(45) Date of publication and mention of the grant of the patent: **09.04.1997**
(21) Application number: 91109379.7
(22) Date of filing: 07.06.1991
(51) Int. Cl.: G06F 17/40, G06F 159/00

(54) **Portable electronic logbook and method of storing and displaying data**
Tragbares elektronisches Tagebuch und Verfahren zur Speicherung und Wiedergabe der Daten
Journal électronique portable et méthode d'emmagasinage et d'affichage des données

(30) Priority: 20.06.1990 US 541061
(43) Date of publication of application: 27.12.1991
(73) Proprietor: Bayer Corporation, Pittsburgh, PA 15219-2502 (US)
(72) Inventor: Bussman, Stephen, Granger, Indiana 46530 (US); Huang, Dijia, Mishawaka, Indiana 46545 (US); Omundson, Dee M., Mishawaka, Indiana 46544 (US); Ruggiero, Joseph E., Goshen, Indiana 46526 (US)
(74) Representative: Dänner, Klaus, Dr.

(56) References cited:
- EP-A- 0 290 683
- GB-A- 2 159 625
- US-A- 4 731 726

## Description

### Field of the Invention

The present invention relates generally to a diabetes information management system and more particularly to a portable electronic logbook storing and displaying information for use by a diabetic patient.

### Description of the Prior Art

Historically a diary or logbook has been used for manually recording various parameters associated with the treatment of a diabetic patient. While such a manual logbook system is generally effective for its intended purpose, entry of the data is time-consuming and then the recorded data is difficult to analyze and use.

U.S. patent 4,731,726 recognizes that most physicians cannot make intelligent treatment decisions by reviewing pages of raw data and that applying data gathered from home glucose monitoring to an insulin adjustment algorithm is too complicated for most patients to follow. This reference discloses a patient-operated glucose monitor and diabetes management system that includes a computer assisted reflectance photometer arranged for measuring and storing blood glucose values at home and for generating recommendations relative to patient insulin dosage based upon the blood glucose data, physician data and other patient data. The disclosed monitor evaluates stored data according to an insulin adjustment algorithm and adjusts an insulin prescription in response to the evaluation. A significant disadvantage of this arrangement is that it effectively does not provide for independent evaluation of the data by the patient.

A portable electronic logbook of the type specified in the preamble of Claim 1 is known from EP-A-0 290 683, for example.

### Summary of the Invention

It is an object of the present invention to provide an electronic logbook that facilitates both self-management skills by a diabetic patient and review by a treating physician.

Among other principal objects of the present invention are to provide an electronic logbook storing and displaying information for use by a diabetic patient; to provide an electronic logbook that is portable, for example, a hand-held device; to provide an electronic logbook having a simple configuration and being economical to manufacture; to provide an electronic logbook including a memory card for program and data storage; and to provide an electronic logbook that overcomes many of the disadvantages of prior art arrangements.

In brief, the objects and advantages of the present invention are achieved by an electronic logbook as claimed in Claim 1.

### Brief Description of the Drawings

The present invention together with the above and other objects and advantages may best be understood from the following detailed description of the embodiment of the invention illustrated in the drawings, wherein:
FIG. 1 is a side perspective view of a first embodiment of a portable electronic logbook in accordance with the present invention;
FIG. 2 is an electrical schematic and block diagram representation of the portable electronic logbook of FIG. 1; and
FIGS. 3-31 are flow charts illustrating the logical steps performed by the portable electronic logbook of FIG. 1.

### Detailed Description of the Preferred Embodiment

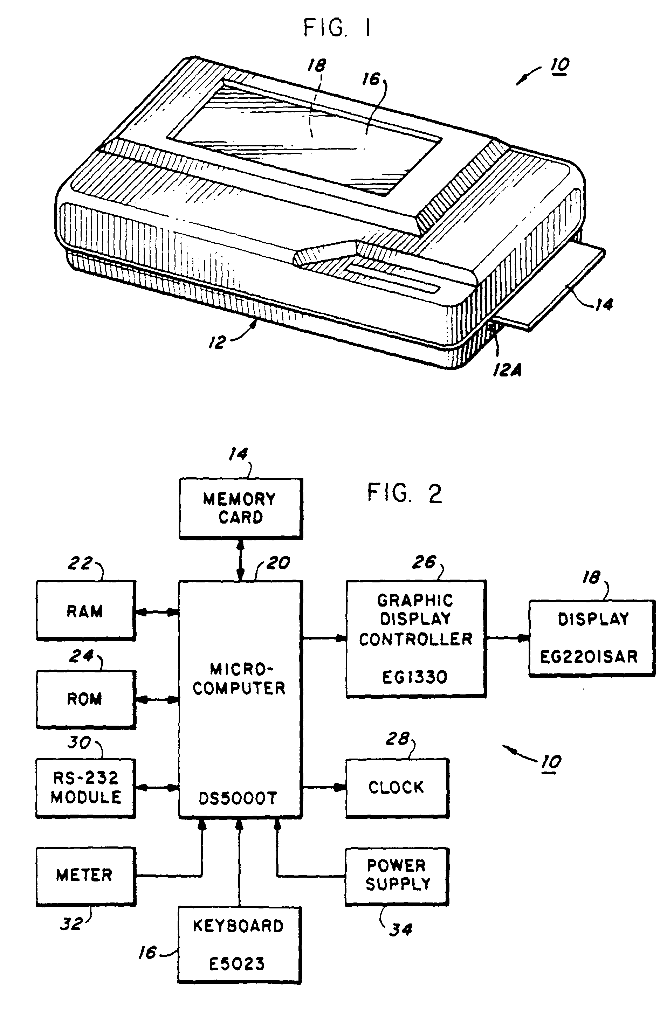

Referring now to the drawings, in FIG. 1 there is illustrated a preferred embodiment of a portable electronic logbook generally designated by the reference character 10. In accordance with important features of the present invention, the electronic logbook 10 is a portable hand-held device adapted for simple and convenient electronically recording various indicators including, for example, such as, blood glucose, insulin, exercise, diet and general health. The electronic logbook 10 includes a relatively large display and a memory card for program and data storage. Substantially immediate feedback, analysis and graphical display of statistical data are provided by the electronic logbook 10 for use by the user or diabetics patient facilitating improved self-management skills while eliminating the need for manual record keeping.

As illustrated in FIG. 1, the portable electronic logbook 10 has a generally rectangular housing 12 including a slot 12A for removably receiving a memory card 14. Patient diabetes data and both system and one or more application programs advantageously are stored by the memory card 14 providing transferable memory. Using the memory card 14 facilitates reconfiguration of the electronic logbook 10 by simply replacing or changing the stored program on the card 14. Typically the memory card 14 includes a plastic card support with one or more integrated circuit chips mounted on the card support for providing a desired type or types of memory, such as electronically erasable/programmable read only memory (EEPROM) and/or random access memory (RAM). A conventional arrangement of connection terminals (not shown) are provided on the memory card 14 to facilitate circuit connection with respective contacts of the electronic logbook 10. Various memory cards utilizing conventional technology such as sold by Mitsubishi Plastics Industries Ltd. or various other commercially available devices can be used for the memory card 14.

A touch panel keyboard 16 for receiving user-input diabetes and selections data is disposed over an LCD display 18 carried by the housing 12. The display 18 provides a sufficient video screen area for displaying alphanumeric characters and graphic images in useful report presentations for the user. For example, a large scale dot matrix graphic display having a dot format of 128 columns x 64 rows and an effective viewing area in inches of 2.890 x 1.528 (7.3 x 3.9 centimeters), such as part number EG2201S-AR sold by Epson Inc. of Torrance, California can be used for the display 18. Keyboard 16 can be provided by a matrix of transparent electrodes formed on a glass-film transparent board, such as a 8 x 7 touch panel array part number E-5023 sold by Epson Inc. The overlying arrangement of the keyboard or touch pad 16 and the display 18 minimizes the required overall dimensions for the housing 12. For example, overall electronic logbook dimensions in inches of 6 x 3.6 x 1.2 (15.2 x 9.1 x 3.0 centimeters) can be provided.

Referring now to FIG. 2, there is shown an electrical schematic and block diagram representation of the portable electronic logbook 10. Among its primary components, the portable electronic logbook 10 further includes a processor device such as a microcomputer 20 coupled for bidirectional communications with the memory card 14 and the keyboard 16. Additional associated external memory devices, such as a random access memory (RAM) 22 and a read only memory (ROM) 24 can be used in conjunction with the microcomputer 20.

Various commercially available microcomputer devices can be used for the microcomputer 20, such as an 8-bit microcontroller device type DS5000T manufactured and sold by Dallas Semiconductor. The microcontroller DS5000T is a complementary metal oxide semiconductor (CMOS) low power device which includes a high speed, nonvolatile static CMOS RAM memory space and nonvolatile internal data registers and key configuration registers. On-chip peripheral functions of the microcontroller DS5000T include a parallel or serial interface for initial loading of an application program, two 16-bit event counter/timers, a full duplex serial Input/Output (I/O) port capable of asynchronous or synchronous operation, 32 parallel I/O lines with possible assignment of 18 parallel I/O lines for an expanded bus function for additional external memory beyond the embedded program/data RAM and a watchdog timer.

A graphic display controller 26, such as a graphic display control part number E-1330 sold by Epson Inc., receives control commands from the microcomputer 20 to control the display 18. An external clock 28 for generating a real-time reference and an RS-232 module 30 for communications with a host system can be provided in conjunction with the microcomputer 20. A meter 32 for blood glucose testing, such as a Glucometer^{R} II device manufactured and sold by Diagnostics Division of Miles Inc. of Elkhart, Indiana, advantageously is used in conjunction with the microcomputer 20. The meter 32 can be an integral component part of the electronic logbook 10. Alternatively, meter 32 can consist of a separate unit. A power supply 34 for the electronic logbook 10 is provided by a rechargeable battery source.

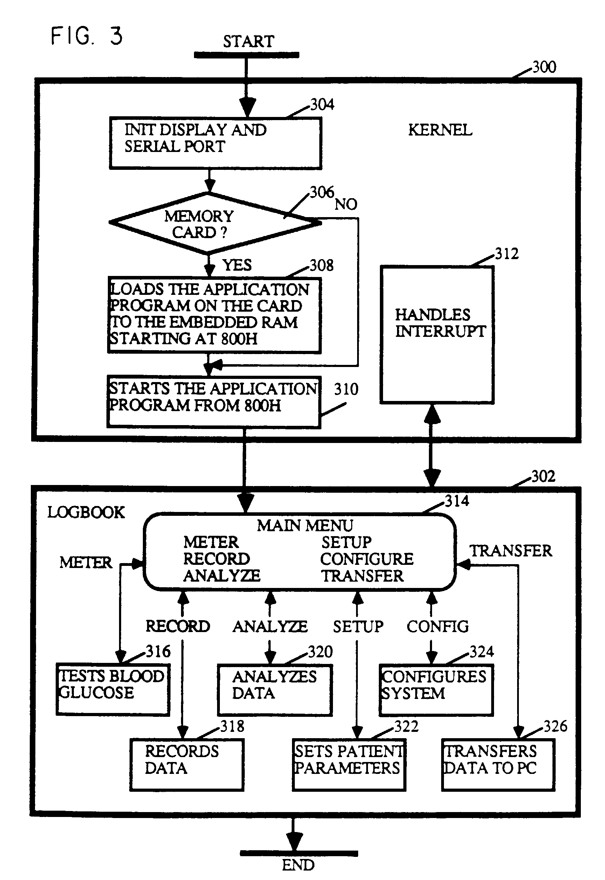

Referring now to FIGS. 3-31, there are shown flow charts illustrating the logical steps performed by the portable electronic logbook 10. In FIG. 3, a simplified representation of a kernel or core of the operating system is indicated at a block 300 and a logbook application program is indicated at a block 302. In a preferred arrangement, the kernel portion of the operating system is located within internal memory in the microcomputer 20, for example, such as the lower 8K of internal RAM. On start-up, initialization of the display and the serial port are performed as indicated at a block 304. Next it is determined whether or not there is a memory card as indicated at a decision block 306. When a memory card 14 is identified at block 306, then the application program on the card is loaded to embedded RAM at a predefined starting address, such as, 800h as indicated at a block 308. Otherwise, when a memory card 14 is not identified at block 306 or after the application program is loaded at block 308, next the application program begins as indicated at a block 310. The kernel of the operating system provides interrupt handling as indicated at a block 312 and low level functions such as, to update the display 18 and to read the touchpad 16.

A main menu first is generated by the logbook application program as indicated at a block 314. User selected functions METER, RECORD, ANALYZE, SETUP, CONFIGURE, TRANSFER are displayed by the main menu screen at predetermined display positions within the display 18. A user input selection is made by the user of the logbook 10 touching a particular displayed functional indicia, such as METER for continuing sequential operations with blood glucose tests as indicated at a block 316.

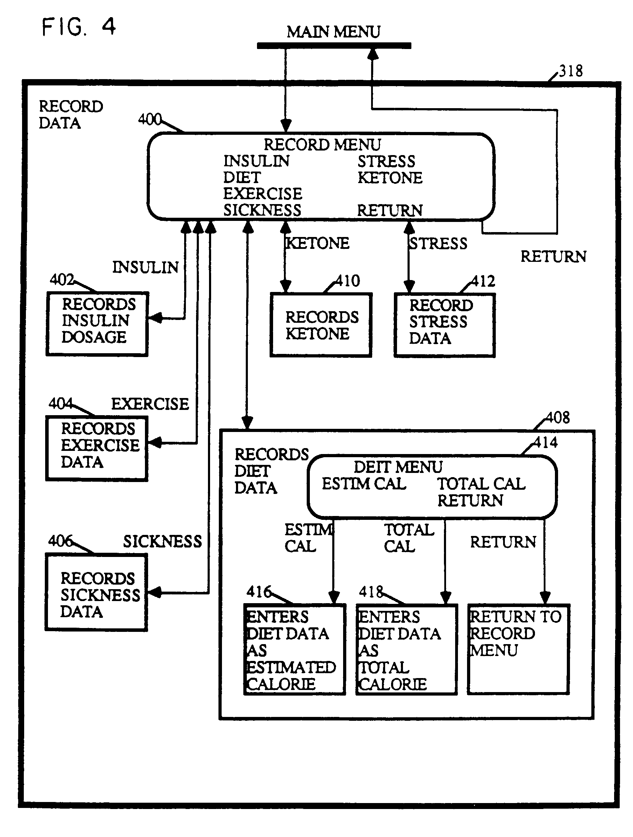

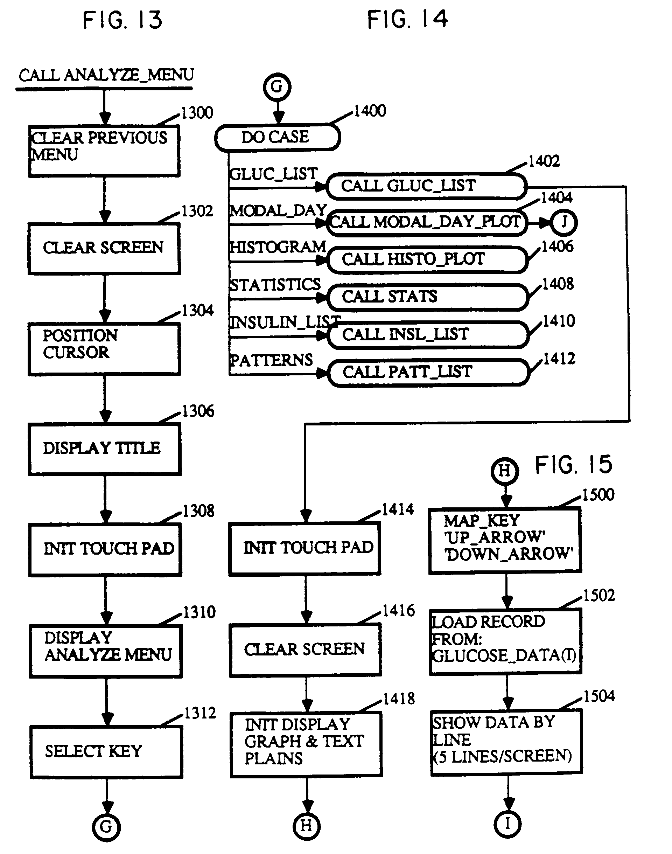

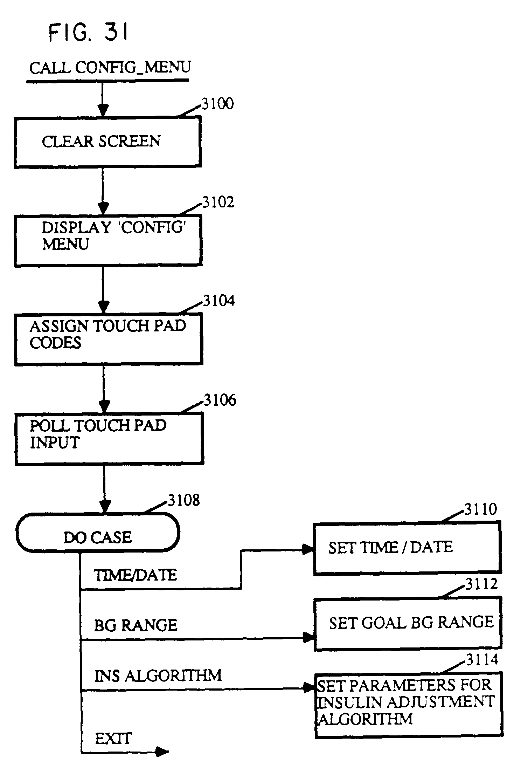

Recording of data is enabled by the user input selection of the RECORD function from the main menu screen 314 with sequential operations continuing as indicated at a block 318 and further illustrated and described with respect to FIGS. 4 and 8-12. Processing and analysis of stored data is enabled by the user input selection of the ANALYSIS function from the main menu screen 314 with sequential operations continuing as indicated at a block 320 and further illustrated and described with respect to FIGS. 13-30. Sequential operations for setting patient parameters are provided by the SETUP function as indicated at a block 322. Sequential operations for setting system parameters are provided by the CONFIG function as indicated at a block 324 and further illustrated and described with respect to FIG. 31. The TRANSFER function as indicated at a block 326 is selected by the user for transferring recorded data to an external computer.

FIG. 4 provides an overview of the sequential logical steps performed following a RECORD user input selection at the main menu block 314. After the data recording program module is called, a first record main menu screen is displayed as indicated at a block 400. Next one of a plurality of patient data categories, such as relating to blood glucose, exercise, diet, insulin, stress, sickness, or ketone is selected by the user touching a particular displayed data category. The sequential operation for the selected data category continues as indicated at blocks 402, 404. 406, 408, 410 and 412 responsive to a respective entry of INSULIN, EXERCISE, SICKNESS, DIET, KETONE and STRESS.

After a data category of DIET is selected via the menu screen at the block 400, a next diet menu screen is displayed as indicated at a block 414. Diet data can be entered as estimated calorie indicated at a block 416 or as total calorie indicated at a block 418 responsive to the user input selection at the block 414.

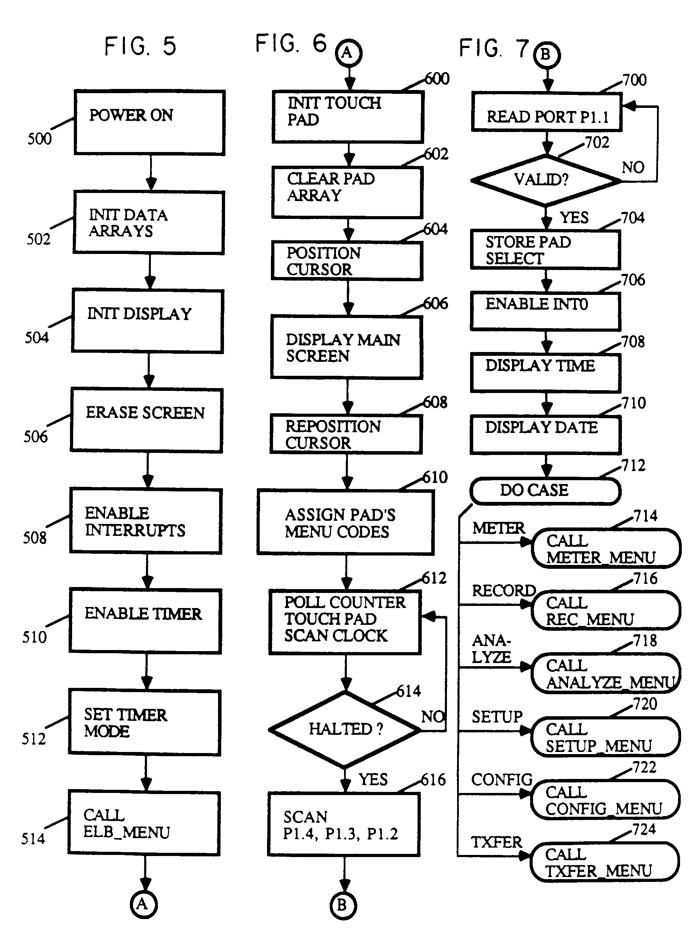

Having reference to FIG. 5, the sequential steps begin with a power on sequence being performed indicated at a block 500. Next the stored data arrays and the display 18 are initialized as indicated at a block 502 and a block 504, respectively. Then an erase screen as indicated at a block 506 is generated. Next interrupts and a timer within the microcomputer or processor 20 are enabled as indicated at a block 508 and a block 510, respectively. A timer mode is set as indicated at a block 512 and then the electronic logbook main menu is called as indicated at a block 514. Sequential operations continue following an entry point A.

Referring to FIG. 6 following the entry point A, the touch pad 16 is initialized as indicated at a block 600 and the pad memory array is cleared or set to zero as indicated at a block 602. Next an internal print text cursor is positioned as indicated at a block 604 and then the main menu screen illustrated in block 312, FIG. 3, is displayed as indicated by a block 606 and the cursor is repositioned as indicated at a block 608. Menu specific codes for the key touch pad 16 are assigned as indicated by a block 610. Next polling of an external counter, the touch pad 16 and scan clock operations are performed to identify a user input selection as indicated by a block 612. When a key is pressed, the output from a high level supply stops a gated oscillator and holds a count value identified as a YES for HALTED operation as indicated by a decision block 614. When a key is not pressed or after when a key is released with a NO decision at the block 614, scanning resumes and continues at the block 612 until a new key is pressed. Otherwise after a key is pressed, then the touch panel decode ports P1.2, P1.3, and P1.4 are scanned as indicated by a block 616. Sequential operations continue following an entry point B in FIG. 7.

In FIG. 7, the sequential steps continue with reading a touch panel decode data port P1.1 as indicated by a block 700. A determination is made whether the port P1.1 input data is valid as indicated by a block 702. When the data is not valid, then the touch panel decode data port P1.1 is read again returning to block 700. Otherwise, when valid data is identified at block 702, then the pad select data is stored as indicated by a block 704. Next the pad interrupt INTO is enabled as indicated at a block 706. Then the current time and date are sequentially displayed as indicated at a block 708 and a block 710. Next a DO CASE sequence begins as indicated by a block 712 with a respective selected one of calling a METER_MENU as indicated by a block 714, a REC_MENU as indicated by a block 716, an ANALYZE_MENU as indicated by a block 718, a SETUP_MENU as indicated by a block 720, a CONFIG_MENU as indicated by a block 722 or a TXFER_MENU as indicated by a block 724.

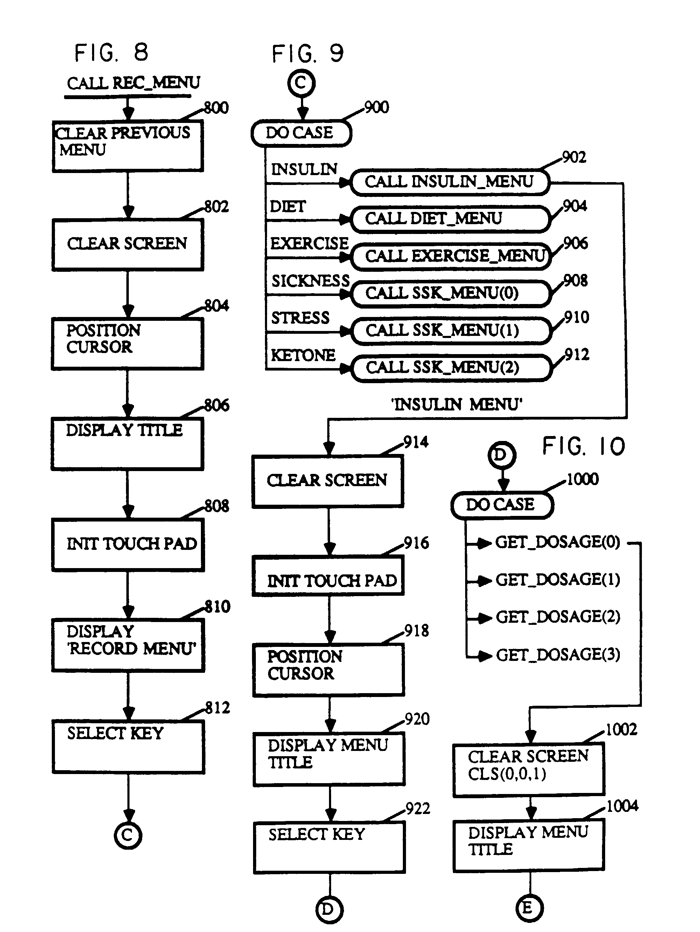

Referring now to FIGS. 8-12, sequential operations for recording of data are illustrated following the call REC_MENU step. The previous menu and screen are cleared as indicated by a block 800 and a block 802, respectively. Then the internal print text cursor is positioned and the record title is displayed as indicated by a block 804 and a block 806. The main record menu shown in block 400 of FIG. 4 is displayed as indicated by a block 810. A user selected key is provided as indicated by a block 812. Sequential operations continue following an entry point C in FIG. 9.

In FIG. 9 following the entry point C, a DO CASE sequence begins as indicated by a block 900 with a respective selected one of calling a INSULIN_MENU as indicated by a block 902, a DIET_MENU as indicated by a block 904, an EXERCISE_MENU as indicated by a block 906, an SSK_MENU(0) as indicated by a block 908, an SSK_MENU(1) as indicated by a block 910 or an SSK_MENU(2) as indicated by a block 912. Next with a selected particular category of patient data menu having been called, then the sequential operations include clearing the screen as indicated at a block 914, initializing the touch pad as indicated at a block 916, positioning the internal text cursor as indicated at a block 918 and displaying the menu title as indicated at a block 920. The user selected key is provided as indicated by a block 920 and the sequential operations continue with FIG. 10 following an entry point D.

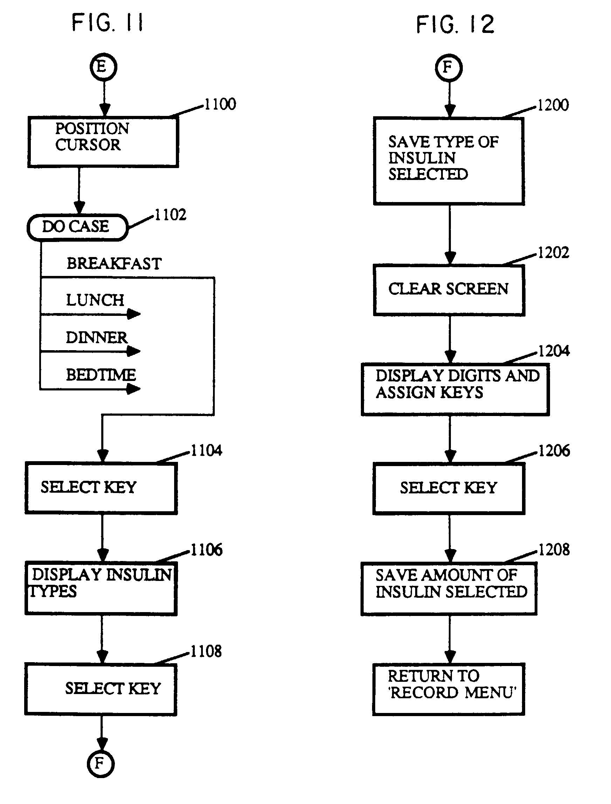

FIG. 10 begins with a DO CASE sequence as indicated by a block 1000 followed with a respective selected one of predefined dosages DOSAGE(0), DOSAGE(1), DOSAGE(2), or DOSAGE(3). Then the screen is cleared and a next menu title is displayed as indicated by a block 1002 and a block 1004. Sequential operations continue following an entry point E in FIG. 11.

In FIG. 11, first the internal text cursor is positioned as indicated by a block 1100, then a DO CASE sequence for a particular meal or bedtime is performed as indicated by a block 1102. The user selected key is provided as indicated by a block 1104 and then the insulin types are displayed as indicated by a block 1106 and the user selected key again is provided as indicated by a block 1108. Sequential operations continue following an entry point F in FIG. 12.

In FIG. 12, first the type of insulin selected is saved as indicated at a block 1200. The screen is cleared as indicated at a block 1202 and then digits are displayed and the touch pad keys are assigned in memory as indicated at a block 1204. A user selected key is identified as indicated at a block 1206 and the amount of insulin selected is saved as indicated at a block 1208. Next the sequential operations return to the main record menu at the beginning of FIG. 8.

FIGS. 13-18 illustrate the sequential operations performed for the ANALYZE functional module of the electronic logbook 10. Referring initially to FIG. 13, the previous menu and screen are cleared as indicated by a block 1300 and a block 1302, respectively. Then the internal print text cursor is positioned and the analyze function title is displayed as indicated by a block 1304 and a block 1306. The touch pad 16 is initialized as indicated by a block 1008. A main analyze menu is displayed as indicated by a block 1310. A user selected key is provided as indicated by a block 1312.

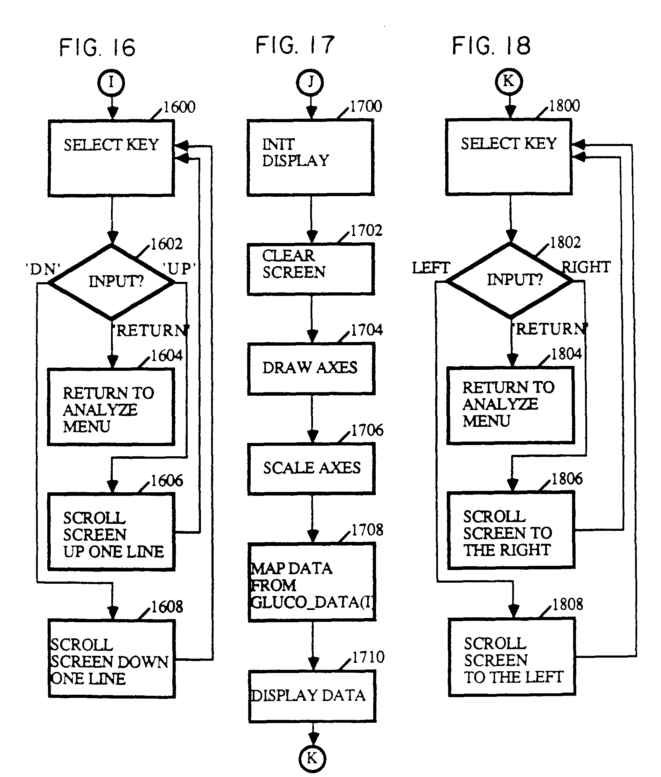

Sequential operations follow an entry point G in FIG. 14 with a DO CASE sequence as indicated by a block 1400. A respective selected one of predefined report generation modules GLUC_LIST, MODAL_DAY_PLOT, HISTO_PLOT, STATS, INSL_LIST or PATT_LIST is called as indicated by the plural blocks 1402, 1404, 1406, 1408, 1410, 1412. After calling the MODAL_DAY_PLOT at block 1404, the sequential operations continue following an entry point J in FIG. 17.

Next after calling the GLUC_LIST module, the touch pad 16 is initialized as indicated at a block 1414, the screen is cleared as indicated at a block 1416 and the display graph and text plains are initialized as indicated at a block 1418. Sequential operations continue following an entry point H in FIG. 15.

In FIG. 15, first an UP_ARROW and DOWN_ARROW are assigned as indicated at a block 1500, a record is loaded from a predefined glucose data array GLUCOSE_DATA(I) as indicated at a block 1502 and a predetermined number of lines of data is displayed as indicated at a block 1504. Sequential operations continue following an entry point I in FIG. 16.

In FIG. 16, the sequential ANALYZE functional operations continue with a select key received and identified as indicated at a block 1600 and a block 1602, respectively. When a RETURN selection is identified at the decision block 1602, then the sequential operations return to the main analyze menu at the block 1310 in FIG. 13 as indicated at a block 1604. When an UP_ARROW selection is identified at the decision block 1602, then the screen display is scrolled up one line as indicated at a block 1606. When a DOWN_ARROW selection is identified at the decision block 1602, then the screen display is scrolled down one line as indicated at a block 1608.

In FIG. 17, the sequential operations to generate a MODAL_DAY_PLOT begin with initializing the display 18 as indicated at a block 1700. Next the screen is cleared as indicated at a block 1702, the axes are drawn as indicated at a block 1704 and the axes are scaled as indicated at a block 1706. Data from the predefined glucose data array GLUCOSE_DATA(I) is mapped and then displayed as indicated by a block 1708 and a block 1710, respectively. Sequential operations continue following an entry point K in FIG. 18.

FIG. 18 continues with a select key received and identified as indicated at a block 1800 and a block 1802, respectively. When a RETURN selection is identified at the decision block 1802, then the sequential operations return to the main analyze menu at the block 1310 in FIG. 13 as indicated at a block 1804. Otherwise when a RIGHT-ARROW or a LEFT-ARROW is identified, then the displayed plot or screen is scrolled to the right or to the left as indicated by a respective one of blocks 1806 and 1808.

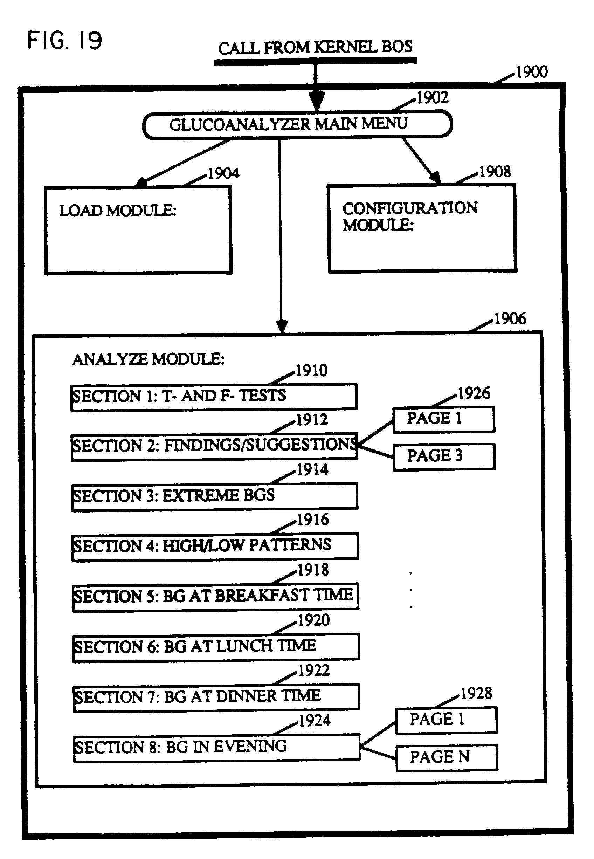

FIGS. 19-30 illustrate a separate application program designated GLUCOANALYZER called from the kernel basic operating system (BOS) as indicated in FIG. 19. The GLUCOANALYZER program indicated in a block 1900 includes a first main menu as indicated in a block 1902. User selections from the main menu at block 1902 include a load module as indicated in a block 1904, an analyze module as indicated in a block 1906, and a configuration module as indicated in a block 1908. The analyze module at block 1906 includes a plurality of data processing SECTIONS 1-8 indicated by blocks 1910, 1912, 1914, 1916, 1918, 1920, 1922, and 1924 for generating a plurality of displayed reports or pages 1-N indicated by blocks 1926 and 1928.

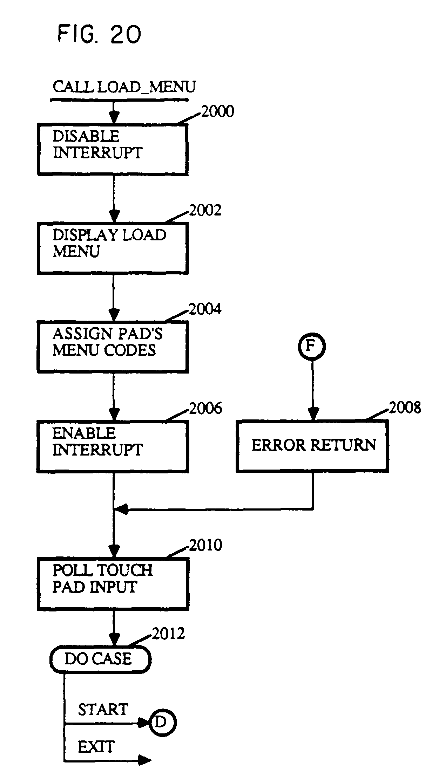

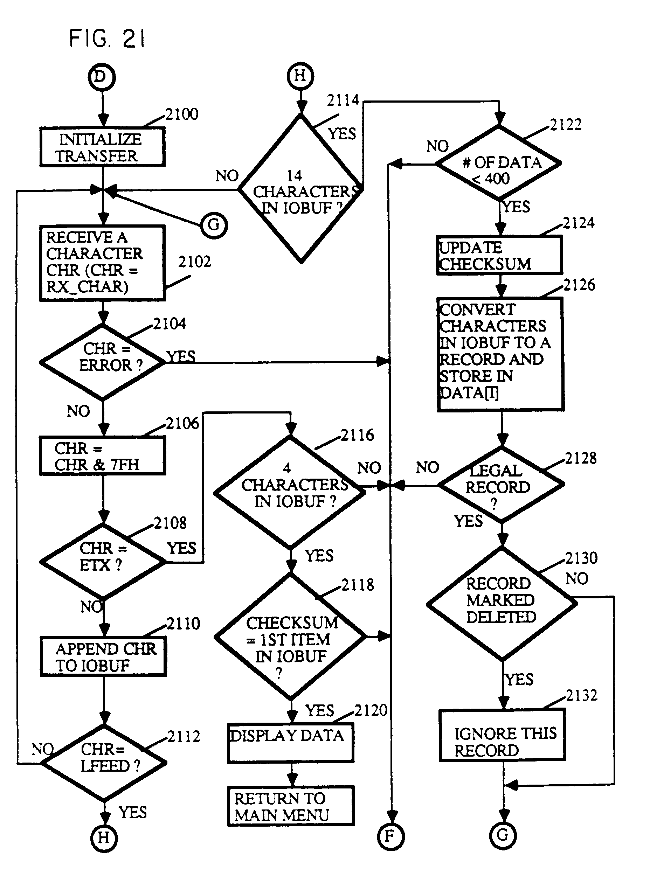

FIGS. 20 and 21 illustrate the steps for loading data from the meter 32 performed by the LOAD module of the GLUCOANALYZER program. Initially the touch pad interrupt INTO is disabled as indicated at a block 2000 and a predefined main load menu is displayed as indicated at a block 2002. Next menu specific codes for the key touch pad 16 are assigned as indicated by a block 2004 and the touch pad interrupt INTO is enabled as indicated by a block 2006. Following an error return after an entry point F from FIG. 21 indicated at a block 2008 or after the touch pad interrupt INTO is enabled at block 2006, the touch pad input is polled as indicated at a block 2010. Next a DO CASE sequence begins as indicated at a block 2012 with a START data transfer following an entry point D in FIG. 21 or to EXIT the data loading module.

Referring now to FIG. 21, following entry point D the data transfer is initialized as indicated by a block 2100. Next a character is received as indicated by a block 2102 and error checking is performed as indicated by a decision block 2104. When an error is identified at block 2104, then the sequential operations return to the error return block 2008 in FIG. 20. Otherwise, testing for an end of text (ETX) character is determined by adding 7fh to the received character as indicated by a block 2106 and then testing for the ETX character as indicated by a decision block 2108.

When the ETX character is identified at block 2108, then whether or not 4 characters are in the I/O buffer IOBUF is determined. When 4 characters are in the I/O buffer IOBUF, then checksum testing is performed as indicated at a block 2118 and if correct, then the data is displayed as indicated at a block 2120 and the sequential operation returns to the main menu at block 1902 in FIG. 19. Otherwise, if the checksum is not identified as correct at block 2118 or if 4 characters are not identified as in the I/O buffer IOBUF at block 2116, then the sequential operations return to the error return block 2008 in FIG. 20.

Otherwise, when the ETX character is not identified at block 2108, then the received character is appended to the characters in the I/O buffer IOBUF as indicated by a block 2110. Next testing for a line feed (LFEED) character is performed as indicated by a block 2112. When a line feed LFEED character is identified at the decision block 2112, then the sequential operations continue following an entry point H in FIG. 21. Otherwise, when a line feed LFEED character is not identified at the decision block 2112, then the sequential operations return to an entry point G in FIG. 21.

Following entry point H, a determination is made of whether or not 14 characters are in the I/O buffer IOBUF as indicated by a decision block 2114. When 14 characters are not in the I/O buffer IOBUF, then the sequential operations return and continue following the entry point G in FIG. 21. Otherwise, when 14 characters are in the I/O buffer IOBUF, then the sequential operations continue with checking the number # of data records as indicated at a block 2122. When determined that the number # of data records is not less than a predetermined number, such as 400, then the sequential operations return to the error return block 2008 following the entry point F in FIG. 20.

Otherwise, when determined that the number # of data records is less than the predetermined number, then the sequential operations continue with an checksum update as indicated by a block 2124. Next the 14 characters in the I/O buffer IOBUF are converted to a record and stored in the predetermined blood glucose data array as indicated at a block 2126. A legal record test is performed as indicated at a decision block 2128. When a legal record is identified, then checking for a marked deleted record is performed as indicated at a decision block 2130. When the marked deleted record is identified, then the record is ignored as indicated at a block 2132. Otherwise, when the marked deleted record is not identified, then the sequential operations again return and continue following the entry point G in FIG. 21.

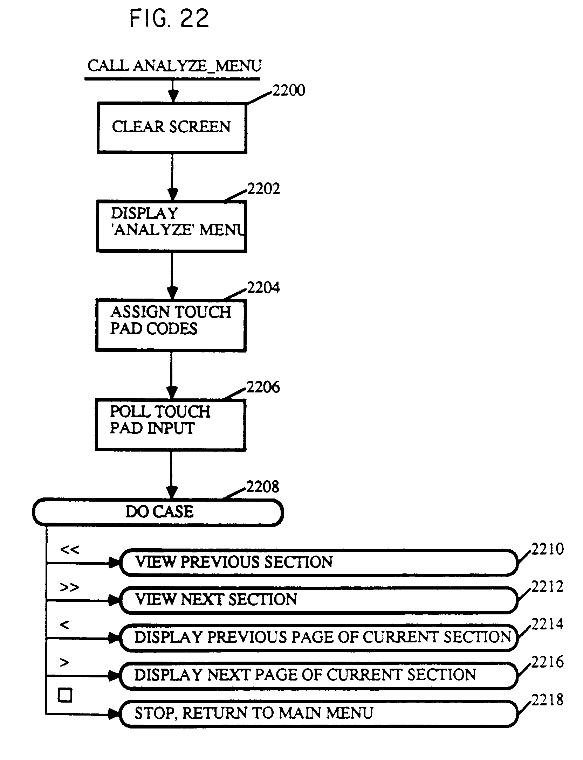

FIGS. 22-30 illustrate the sequential operations of the analyze module shown in block 1906 in FIG. 19. Having reference to FIG. 22, the screen is cleared and the main analyze menu screen is displayed as indicated by a block 2200 and a block 2202, respectively. Then menu specific codes are assigned to the touch pad 16 as indicated by a block 2204. Next the touch pad input is polled as indicated at a block 2206. Next a DO CASE sequence begins as indicated at a block 2208 with an identification of a user selection of VIEW PREVIOUS SECTION, VIEW NEXT SECTION, DISPLAY PREVIOUS PAGE OF CURRENT SECTION, DISPLAY NEXT PAGE OF CURRENT SECTION and STOP, RETURN TO MAIN MENU as indicated by a plurality of blocks 2210, 2212, 2214, 2216 and 2218.

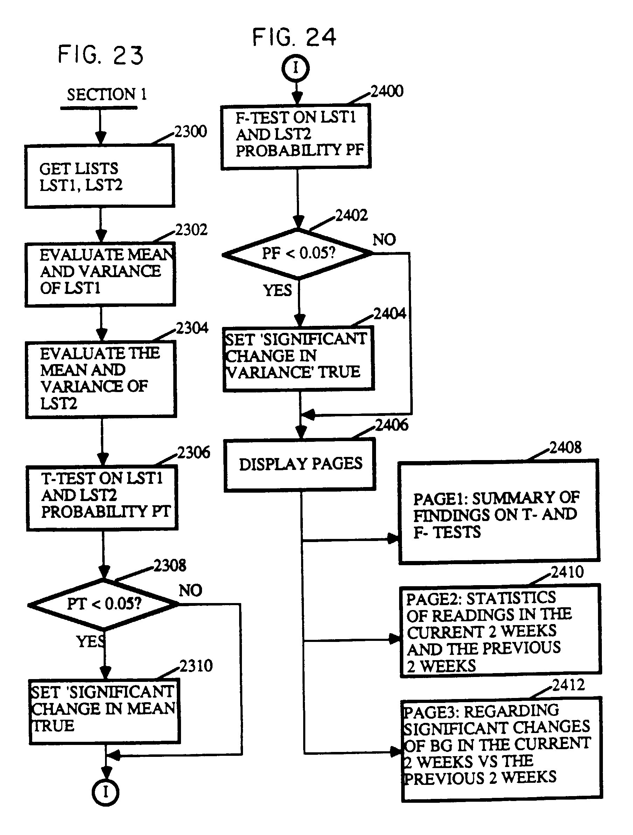

FIG. 23 illustrates the sequential steps of the data processing SECTION 1: T- and F-tests in block 1910 in FIG. 19. As indicated in a block 2300, operations start with a subroutine to get lists LST1, LST2 from blood glucose data array of the current 2 weeks LST1 and the previous 2 weeks LST2. Next the mean and variance of the data list LST1 is evaluated as indicated at a block 2302. Then the mean and variance of the data list LST2 is evaluated as indicated at a block 2304. T-test calculations are made for the data lists LST1, LST2 and a probability PT is determined as indicated at a block 2306. The calculated probability PT is compared with a predetermined threshold value, such as 0.05, as is indicated at a block 2308, in order to identify a significant change in the mean. When the calculated probability PT is less than the predetermined threshold value, then a bit representing a significant change in the mean is set true as indicated at a block 2310. Otherwise, when the calculated probability PT is not less than the predetermined threshold value or after the significant change in the mean is set true, then the sequential operations continue following an entry point I in FIG. 24.

In FIG. 24, first F-test calculations are made for the data lists LST1, LST2 and a probability PF is determined as indicated at a block 2400. The calculated probability PF is compared with a predetermined threshold value, such as 0.05, as is indicated at a block 2402, in order to identify a significant change in the variance. When the calculated probability PF is less than the predetermined threshold value, then a bit representing a significant change in the variance is set true as indicated at a block 2404. Otherwise, when the calculated probability PF is not less than the predetermined threshold value or after the significant change in the variance is set true, then the sequential operations continue with a DISPLAY PAGES routine as indicated at a block 2406. A first report or PAGE 1 displays a summary of findings on the T- and F-tests as indicated at a block 2408. A second report or PAGE 2 displays statistics of readings in the current and previous 2 weeks as indicated at a block 2410. A third report or PAGE 3 displays a comparison of significant changes in blood glucose for the current and previous 2 weeks as indicated at a block 2412.

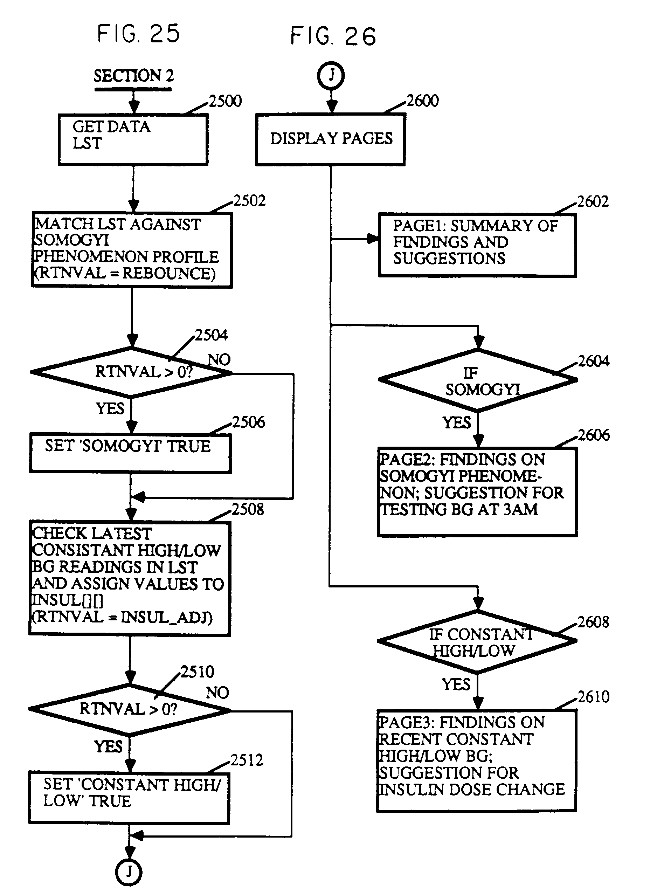

FIGS. 25-26 illustrates the sequential steps of the data processing SECTION 2: FINDINGS/SUGGESTIONS in block 1912 in FIG. 19. As indicated in a block 2500, operations start with a subroutine to get a data list LST from blood glucose data array of the current 7 days. Then the data list LST is matched against a Somogyi phenomenon profile (RTNVAL = REBOUNCE) as indicated at a block 2502. Next it is determined if RTNVAL is greater than zero as indicated at a block 2504. When RTNVAL is greater than zero, the SOMOGYI bit is set true as indicated at a block 2506. Otherwise, when RTNVAL is not greater than zero or after the SOMOGYI bit is set true, then the latest consistent high or low blood glucose BG readings of the data list LST are checked and values are assigned to an insulin data array (RTNVAL = INSUL_ADJ) as indicated at a block 2508. Next it is determined if RTNVAL is greater than zero as indicated at a block 2510. When RTNVAL is greater than zero, the CONSTANT HIGH/LOW bit is set true as indicated at a block 2512. Sequential operations continue following an entry point J in FIG. 26.

In FIG. 26, the sequential operations continue with a DISPLAY PAGES routine as indicated at a block 2600. A first report or PAGE 1 displays a summary of findings and suggestions as indicated at a block 2602. When the SOMOGYI true is identified as indicated by a decision block 2604, then a second report or PAGE 2 displays findings on Somogyi phenomenon and a suggestion for blood glucose testing at 3AM as indicated at a block 2606. When the CONSTANT HIGH/LOW true is identified as indicated by a decision block 2608, then a next report or PAGE 3 displays findings on constant high or low blood glucose and a suggestion for an insulin dose change as indicated at a block 2610.

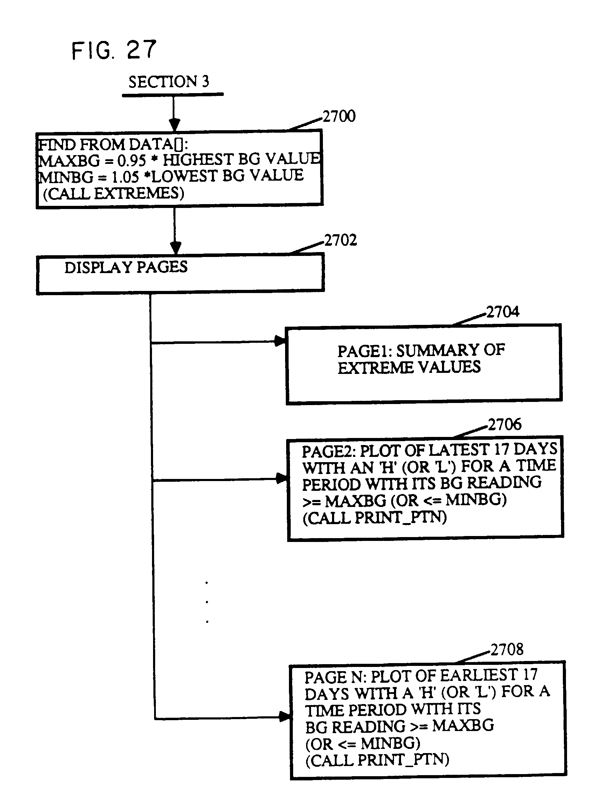

FIG. 27 illustrates the sequential steps of the data processing SECTION 3: EXTREME BGS in block 1914 in FIG. 19. The sequential operations begin with finding the highest and lowest blood glucose values from the blood glucose data array as indicated at a block 2700. Next the DISPLAY PAGES routine is performed as indicated at a block 2702. A first report or PAGE 1 displays a summary of extreme values as indicated at a block 2704. A second report or PAGE 2 is generated by calling a plot generating routine (CALL PRINT-PTN) for displaying a plot of high or low extreme values with its BG reading using the latest 17 days as indicated at a block 2706. A last report or PAGE N displays a similar plot for the earliest 17 days as indicated at a block 2708.

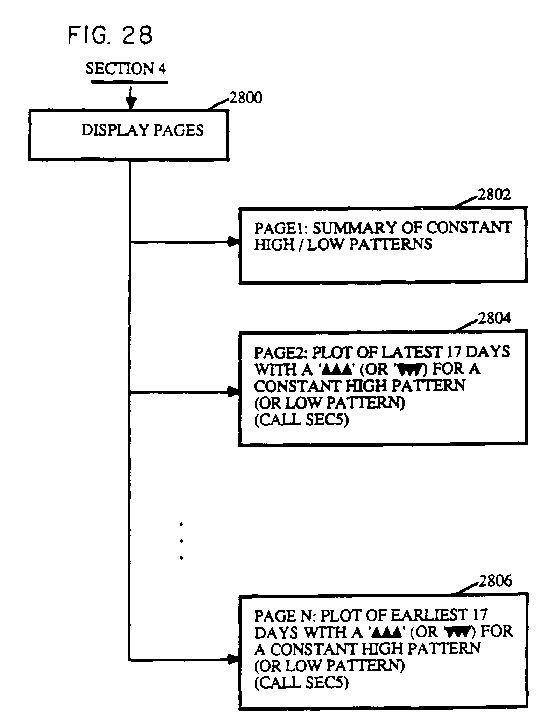

FIG. 28 illustrates the sequential steps of the data processing SECTION 4: HIGH/LOW PATTERNS in block 1916 in FIG. 19. The sequential operations begin with a DISPLAY PAGES routine as indicated at a block 2800. A first report or PAGE 1 displays a summary of constant high/low patterns as indicated at a block 2802. A second report or PAGE 2 is generated by calling a plot generating routine (CALL SEC5) for displaying a plot of constant high or constant low readings using either up-triangle symbols for high or inverted-triangle symbols for low readings in the latest 17 days as indicated at a block 2806. A last report or PAGE N displays a similar plot for the earliest 17 days as indicated at a block 2806.

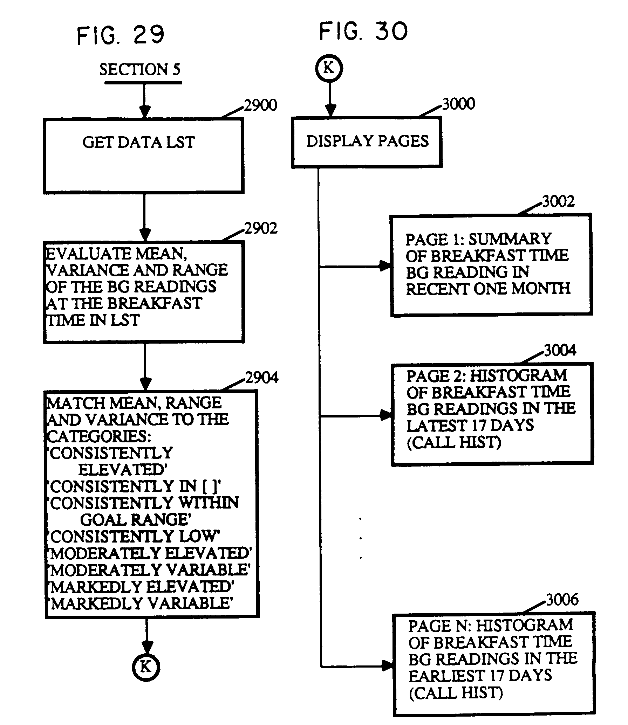

FIGS. 29-30 illustrates the sequential steps of the data processing SECTION 5: BG AT BREAKFAST TIME in block 1918 in FIG. 19. As indicated in a block 2900, operations start with a subroutine to get a data list LST from blood glucose data array of the latest 30 days. The mean, variance and range of the blood glucose readings at the breakfast time is evaluated as indicated at a block 2902. Next the calculated mean, range and variance is matched to a plurality of categories including consistently elevated, consistently in [i.e., 300 to 400 mg/dL (milligrams/deciliter)], consistently within goal range [i.e., 80 to 150 mg/dL], consistently low, moderately elevated, moderately variable, markedly elevated and markedly variable as indicated at a block 2904. Sequential operations continue following an entry point K in FIG. 30.

In FIG. 30, the sequential operations continue with a DISPLAY PAGES routine as indicated at a block 3000. A first report or PAGE 1 displays a summary of breakfast time blood glucose readings for the recent one month as indicated at a block 3002. A second report or PAGE 2 is generated by performing a histogram generating routine (CALL HIST) and displays a histogram of breakfast time blood glucose readings for the recent 17 days as indicated at a block 3004. A last report or PAGE N is generated by performing the routine CALL HIST and displays a histogram of breakfast time blood glucose readings for the earliest 17 days as indicated at a block 3006.

A similar sequence as described for the SECTION 5 is performed for SECTIONS 6, SECTION 7 and SECTION utilizing a data array for a respective one of lunch time, dinner time or evening.

FIG. 31 illustrates the sequential steps of the configuration module illustrated at block 1908 in FIG. 19. First the screen is cleared as indicated by a block 3100. Then the configuration CONFIG main menu is displayed as indicated by a block 3102. The touch pad 16 is assigned menu specific codes as indicated by a block 3104 and then is polled as indicated by a block 3106. Then sequential operations continue with a DO CASE sequence as indicated by a block 3108. A respective selected one of predefined system parameters are then updated or set of SET TIME/DATE as indicated at a block 3110, SET GOAL BG RANGE as indicated at a block 3112, and SET PARAMETERS FOR INSULIN ADJUSTMENT ALGORITHM as indicated at a block 3114.

## Claims

1. A portable electronic logbook (10) for storing and displaying diabetes patient information comprising:
a housing (12) having a slot (12A);
user input means (16) carried by said housing for entering a plurality of user input selections;
memory means (14) for storing patient and program data;
processor means (20) coupled to said memory means (14) and said user input means (16); said processor means including program means (302) for generating a main menu screen (314), said main menu screen including a plurality of predetermined indicia corresponding to a subplurality of said user input selections, program means for generating a plurality of functional menu screens, program means responsive to said user input means for identifying a user input selection,
display means (18) carried by said housing (12) operatively driven by said processor means for selectively displaying said main menu screen, for selectively displaying one of said plurality of functional menu screens responsive to a user input selection entered from said user input means,
characterized in that
said memory means is a memory card (14) removably entered into said housing slot (12A);
said user input means (16) being a substantially transparent touch board disposed in spaced relationship above said display means;
said program means are responsive to said user input selection identifying means for selectively accessing said stored patient data and for generating a user selected report; and
said display means (18) selectively display said user selected report generated by said processor means (20).

2. A portable electronic logbook (10) as recited in claim 1 wherein said housing (12) is a small, hand-held housing having said card receiving slot (12A) for removably receiving said memory card (14).

3. A portable electronic logbook (10) as recited in claim 1 wherein said selectively displayed user selected report includes a user selected plot of patient data relating to blood glucose, exercise, diet, insulin, stress, sickness, or ketone.

4. A portable electronic logbook (10) as recited in claim 1 wherein said user selected displayed patient data includes displaying a left arrow indicia and a right arrow indicia; means for identifying a user input selection of said displayed left arrow indicia or said right arrow indicia; and means responsive to said identified user input selection for displaying a plot.

5. A portable electronic logbook (10) as recited in claim 1 wherein one of said plurality of functional menu screens corresponds to a METER function for performing a blood glucose test.

6. A portable electronic logbook (10) as recited in claim 1 wherein one of said plurality of functional menu screens corresponds to a RECORD function for recording patient data.

7. A portable electronic logbook (10) as recited in claim 1 wherein one of said plurality of functional menu screens corresponds to a SETUP function for setting patient parameters.

8. A portable electronic logbook (10) as recited in claim 1 wherein one of said plurality of functional menu screens corresponds to a CONFIGURE function for recording patient specific data including goal blood glucose range values and parameters for an insulin adjustment algorithm.

9. A portable electronic logbook (10) as recited in claim 1 wherein one of said plurality of functional menu screens corresponds to a TRANSFER function for transferring recorded data to an external computer.

10. A portable electronic logbook (10) as recited in claim 1 wherein one of said plurality of functional menu screens corresponds to an ANALYZE function for processing patient data and for generating said desired report.

## Patentansprüche

1. Ein tragbares elektronisches Tagebuch zur Speicherung und Wiedergabe von Diabetes-Patienteninformation, umfassend:
- ein Gehäuse (12) mit einem Schlitz (12A);
- eine durch das Gehäuse getragene Benutzereingabevorrichtung (16) zum Eingeben einer Vielzahl von Benutzereingabeauswahlen;
- eine Speichervorrichtung (14), zum Speichern von Patientenund Programmdaten;
- eine Prozessorvorrichtung (20), die mit der Speichervorrichtung (14) und der Benutzereingabevorrichtung (16) verbunden ist; wobei die Prozessorvorrichtung eine Programmvorrichtung (302) zum Erzeugen eines Hauptmenüschirmes (314) einschließt, wobei der Hauptmenüschirme eine Vielzahl von im voraus festgelegten Zeichen einschließt, die einer Untervielzahl der Benutzereingabeauswahlen entsprechen, eine Programmvorrichtung zum Erzeugen einer Vielzahl von Funktionsmenüschirmen, eine auf die Benutzereingabevorrichtung ansprechende Programmvorrichtung zum Identifizieren einer Benutzereingabeauswahl;
- eine vom Gehäuse (12) getragene Anzeigevorrichtung (18), die betriebsmäßig durch die Prozessorvorrichtung getrieben wird, um den Hauptmenüschirm selektiv anzuzeigen, um einen der Vielzahl von Funktionsmenüschirmen, ansprechend auf eine von der Benutzereingabevorrichtung eingegebene Benutzereingabe, selektiv anzuzeigen,
dadurch gekennzeichnet, daß
- die Speichervorrichtung eine entfernbar in den Gehäuseschlitz (12A) eingeführte Speicherkarte (14) ist;
- die Benutzereingabevorrichtung (16) ein im wesentlichen durchsichtiges Berührungsfeld ist, das in einer beabstandeten Beziehung über der Anzeigevorrichtung angeordnet ist;
- die Programmvorrichtung auf die Benutzereingabeauswahl-Identifizierungsvorrichtung anspricht, um auf die gespeicherten Patientendaten selektiv zuzugreifen, und um einen vom Benutzer ausgewählten Bericht zu erzeugen; und
- die Anzeigevorrichtung (18) den durch die Prozessorvorrichtung (20) erzeugten benutzerausgewählten Bericht selektiv anzeigt.

2. Ein tragbares elektronisches Tagebuch (10) nach Anspruch 1, worin das Gehäuse (12) ein kleines handgehaltenes Gehäuse ist, mit dem kartenaufnehmenden Schlitz (12A), um die Speicherkarte (14) entfernbar aufzunehmen.

3. Ein tragbares elektronisches Tagebuch (10) nach Anspruch 1, worin der selektiv angezeigte, vom Benutzer ausgewählte Bericht eine vom Benutzer ausgewählte Darstellung von Patientendaten bezüglich Blutglukose, Übungen, Insulin, Diät, Streß, Krankheit oder Keton einschließt.

4. Ein tragbares elektronisches Tagebuch (10) nach Anspruch 1, worin die vom Benutzer ausgewählten angezeigten Patientendaten ein Anzeigen eines linken Pfeilzeichens und eines rechten Pfeil zeichens einschließen; eine Vorrichtung zum Identifizieren einer Benutzereingabeauswahl des angezeigten linken Pfeilzeichens oder rechten Pfeilzeichens; und eine Vorrichtung, die auf die identifizierte Benutzereingabeauswahl anspricht, um eine Darstellung anzuzeigen.

5. Ein tragbares elektronisches Tagebuch (10) nach Anspruch 1, worin einer der Vielzahl von Funktionsmenüschirmen einer MESSEN Funktion zum Durchführen eines Blutglukosetests entspricht.

6. Ein tragbares elektronisches Tagebuch (10) nach Anspruch 1, worin einer der Vielzahl von Funktionsmenüschirmen einer AUFZEICHEN Funktion zum Aufzeichnen von Patientendaten entspricht.

7. Ein tragbares elektronisches Tagebuch (10) nach Anspruch 1, worin einer der Vielzahl von Funktionsmenüschirmen einer EINRICHTEN Funktion zum Setzen von Patientenparametern entspricht.

8. Ein tragbares elektronisches Tagebuch (10) nach Anspruch 1, worin einer der Vielzahl von Funktionsmenüschirmen einer KONFIGURIEREN Funktion zum Aufzeichnen von für den Patienten spezifischen Daten entspricht, einschließlich Blutglukose-Zielbereich-Werten und Parametern für einen Insulin-Einstellalgorithmus.

9. Ein tragbares elektronisches Tagebuch (10) nach Anspruch 1, worin einer der Vielzahl von Funktionsmenüschirmen einer ÜBERTRAGEN Funktion zum Übertragen von Daten zu einem externen Computer entspricht.

10. Ein tragbares elektronisches Tagebuch (10) nach Anspruch 1, worin einer der Vielzahl von Funktionsmenüschirmen einer ANALYSIEREN Funktion zum Verarbeiten von Patientendaten und zum Erzeugen des gewünschten Berichts entspricht.

## Revendications

1. Carnet journalier électronique portatif (10) pour stocker et afficher des informations concernant un patient diabétique, comportant :
un boîtier (12) ayant une fente (12A) ;
un moyen (16) d'entrée pour l'utilisateur, porté par ledit boîtier pour l'introduction de plusieurs sélections d'entrée de l'utilisateur ;
un moyen à mémoire (14) destiné à stocker des données du patient et d'un programme ;
un moyen à processeur (20) couplé audit moyen à mémoire (14) et audit moyen (16) d'entrée de l'utilisateur ; ledit moyen à processeur comprenant un moyen à programme (302) destiné à générer un écran (314) de menu principal, ledit écran de menu principal comprenant une pluralité de signes prédéterminés correspondant à un sous-ensemble desdites sélections d'entrée de l'utilisateur, un moyen à programme destiné à générer une pluralité d'écrans de menus fonctionnels, un moyen à programme qui, en réponse audit moyen d'entrée de l'utilisateur, est destiné à identifier une sélection d'entrée de l'utilisateur,
un moyen d'affichage (18) porté par ledit boîtier (12), attaqué fonctionnellement par ledit moyen à processeur pour afficher sélectivement ledit écran de menu principal, pour afficher sélectivement l'un de ladite pluralité d'écrans de menus fonctionnels en réponse à une sélection d'entrée de l'utilisateur introduite à partir dudit moyen d'entrée de l'utilisateur,
caractérisé en ce que
ledit moyen à mémoire est une carte à mémoire (14) introduite de façon amovible dans ladite fente (12A) du boîtier ;
ledit moyen d'entrée (16) pour l'utilisateur étant un panneau tactile sensiblement transparent disposé à distance au-dessus dudit moyen d'affichage ;
lesdits moyens à programmes réagissent audit moyen d'identification de sélection d'entrée de l'utilisateur pour accéder sélectivement auxdites données stockées du patient pour générer un rapport choisi par l'utilisateur ; et
ledit moyen d'affichage (18) affiche sélectivement ledit rapport choisi par l'utilisateur, généré par ledit moyen à processeur (20).

2. Carnet journalier électronique portatif (10) selon la revendication 1, dans lequel ledit boîtier (12) est un petit boîtier tenu à la main ayant ladite fente (12A) de réception de carte pour recevoir de façon amovible ladite carte (14) à mémoire.

3. Carnet journalier électronique portatif (10) selon la revendication 1, dans lequel ledit rapport sélectionné par l'utilisateur est affiché sélectivement en un graphique, choisi par l'utilisateur, de données du patient concernant le glucose du sang, l'exercice, l'alimentation, l'insuline, la tension, une maladie, ou une cétone.

4. Carnet journalier électronique portatif (10) selon la revendication 1, dans lequel lesdites données du patient affichées, sélectionnées par l'utilisateur, comprennent l'affichage d'un signe de flèche à gauche et d'un signe de flèche à droite ; des moyens pour identifier une sélection d'entrée par l'utilisateur dudit signe de flèche à gauche affiché ou dudit signe de flèche à droite ; et des moyens qui, en réponse à ladite sélection d'entrée par l'utilisateur, identifié, sont destinés à afficher un graphique.

5. Carnet journalier électronique portatif (10) selon la revendication 1, dans lequel l'un de ladite pluralité d'écrans de menus fonctionnels correspond à une fonction MESURE pour effectuer un test de glucose du sang.

6. Carnet journalier électronique portatif (10) selon la revendication 1, dans lequel l'un de ladite pluralité d'écrans de menu fonctionnels correspond à une fonction ENREGISTREMENT pour enregistrer les données du patient.

7. Carnet journalier électronique portatif (10) selon la revendication 1, dans lequel l'un de ladite pluralité d'écrans de menus fonctionnels correspond à une fonction PARAMETRAGE pour établir des paramètres pour le patient.

8. Carnet journalier électronique portatif (10) selon la revendication 1, dans lequel l'un de ladite pluralité d'écrans de menus fonctionnels correspond à une fonction CONFIGURATION pour enregistrer des données spécifiques du patient comprenant des valeurs recherchées de plage de glucose du sang et des paramètres pour un algorithme d'ajustement de l'insuline.

9. Carnet journalier électronique portatif (10) selon la revendication 1, dans lequel l'un de ladite pluralité d'écrans de menus fonctionnels correspond à une fonction TRANSFERT pour transférer des données enregistrées vers un calculateur extérieur.

10. Carnet journalier électronique portatif (10) selon la revendication 1, dans lequel l'un de ladite pluralité d'écrans de menus fonctionnels correspond à une fonction ANALYSE pour le traitement de données du patient et pour la génération dudit rapport souhaité.
